# EUROPÄISCHE PATENTSCHRIFT

(11) **EP 0 771 796 B1**
(45) Veröffentlichungstag und Bekanntmachung des Hinweises auf die Patenterteilung: **19.06.2002**
(21) Anmeldenummer: 96117262.4
(22) Anmeldetag: 28.10.1996
(51) Int. Cl.: C07D 401/06, C07D 417/06, C07D 403/06, C07D 413/06, C07D 409/06, G03C 1/73

(54) **Substituierte s-Triazine und Verfahren zu ihrer Herstellung**
Substituted S-triazines and process for their preparation
S-triazines substituées et procédé pour leur préparation

(30) Priorität: 02.11.1995 DE 19540796
(43) Veröffentlichungstag der Anmeldung: 07.05.1997
(73) Patentinhaber: AGFA-GEVAERT, 2640 Mortsel (BE)
(72) Erfinder: Canestri, Giuseppe, Prof. Dr., 38064 Folgaria (TN) (IT)
(74) Vertreter: Van Ostaeyen, Marc Albert Jozef

(56) Entgegenhaltungen:
- EP-A- 0 359 430
- DE-A- 4 435 791
- DE-A- 4 438 137
- DATABASE WPI Week 9218 Derwent Publications Ltd., London, GB; AN 92-145276 XP002021757 & JP-A-04 081 761 (KONICA CORP.; MITSUBISHI KASEI CORP.) , 16.März 1992
- DATABASE WPI Week 9304 Derwent Publications Ltd., London, GB; AN 93-033452 XP002021758 & JP-A-04 362 643 (KONICA CORP.) , 15.Dezember 1992
- DATABASE WPI Week 9644 Derwent Publications Ltd., London, GB; AN 96-439587 XP002021759 & JP-A-08 217 813 (TOYO INK MFG. CO., LTD.) , 27.August 1996

## Beschreibung

Die Erfindung betrifft chromophor-substituierte Trihalogenmethyl-s-triazine und ein Verfahren zu ihrer Herstellung.

Verbindungen dieser Art und Verfahren zu ihrer Herstellung sind bekannt. Aus der GB-A 1 388 492 sind Triazine bekannt, die mit einer oder zwei Trihalogenmethylgruppe(n) und - über eine Brückengruppe -[CH=CH]ₙ- (wobei n eine ganze Zahl von 1 bis 3 bedeutet) - mit einer heterocyclischen Gruppe, nämlich einer Benzooxazol-2-ylidenmethyl- oder Benzothiazol-2-ylidenmethylgruppe, substituiert sind. Der Benzooxazol- oder Benzothiazolring kann am Stickstoffatom (C₁-C₆)-Alkyl- oder Phenylsubstituenten tragen. Die zweite Trihalogenmethylgruppe kann durch eine Amino-, Anilino-, Diphenylamino- oder (C₁-C₆)-Alkoxygruppe ersetzt sein. Diese Verbindungen finden als Photoinitiatoren in strahlungsempfindlichen Gemischen Anwendung. Man kann sie in einer Knoevenagel-Reaktion aus einem trihalogenmethyl-substituierten s-Triazin und einem 2-Formyl-, 2-(3-Oxopropenyl)- oder 2-(5-Oxopenta-1,3-dienyl)-oxazolinium- oder -thiazoliniumsalz herstellen. Als Anion können diese Salze Chlorid, Bromid, Iodid, Sulfat, Toluolsulfonat oder Ethoxyethylsulfat enthalten.

Gemäß US-A 4 239 850 erhöht sich die Strahlungsempfindlichkeit von chromophor-substituierten Trihalogenmethyls-triazinen, wenn man sie im Gemisch mit einem Keton einsetzt, das mit einem Stickstoff-Heterocyclus substituiert ist. Das substituierte Keton hat die Formel 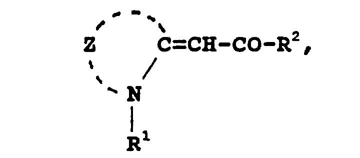 worin
- R¹: eine gegebenenfalls substituierte Alkylgruppe und
- R²: eine Alkyl- oder Arylgruppe bedeutet.

Polymerisationskatalysatoren sind auch aus der EP-A 438 123 bekannt. Diese enthalten einen im nahen Infrarot absorbierenden kationischen Farbstoff und einen borhaltigen Sensibilisator. Cyanin, Triarylmethan, Aminium und Diimonium werden als kationische Farbstoffe genannt, die Anionen wie Halogenid, Perchlorat, Hexafluorophosphat, Tetrafluoroborat, Hexafluoroantimonat, Methansulfonat, Trifluormethansulfonat, Benzolsulfonat, Toluolsulfonat, Hydroxybenzolsulfonat, Chlorbenzolsulfonat und quaternäres Borat enthalten. Als Sensibilisator wird ein Komplex aus quaternärem Ammonium und Borat verwendet, wie Tetramethylammonium-n-butyltriphenylborat oder Tetraethylammonium-tetrabutylborat.

Aus der JP-A 04-081 761 ist eine lichtempfindliche Zusammensetzung zur Herstellung von vorsensibilisierten Druckplatten bekannt. Diese kann ein 2-[2-(4,6-Bis-trichlormethyl-s-triazin-2-yl)-vinyl]-1-methyl-chinoliniumsalz, ein 4-[2-(4,6-Bis-trichlormethyl-s-triazin-2-yl)-vinyl]-1-methyl-pyridiniumsalz oder ähnliche Salze enthalten. Bei Bestrahlung setzen diese Verbindungen eine Säure frei. Die Zusammensetzung enthält in der Regel zusätzlich noch einen Novolak.

Aus der JP-A 04-362 643 ist eine lichtempfindliche Zusammensetzung bekannt, in der die Säure freisetzenden Salze mit säurespaltbaren Verbindungen kombiniert werden.

Es ist die Aufgabe dieser Erfindung, Sensibilisatoren mit verbesserter Strahlungsempfindlichkeit bei Wellenlängen von 330 bis 720 nm bereitzustellen.

Diese Aufgabe wird gelöst durch Verbindungen der Formel I 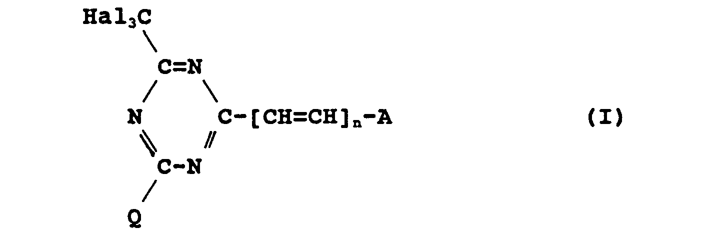 worin
- n: eine ganze Zahl von 1 bis 3,
- Hal: ein Halogenatom,
- Q: CCl₃, CBr₃, NH₂, NHR, NRR' oder OR, wobei R und R' unabhängig voneinander eine (C₁-C₆)-Alkylgruppe oder eine Phenylgruppe bedeuten, und
- A: ein Rest einer der folgenden Formeln 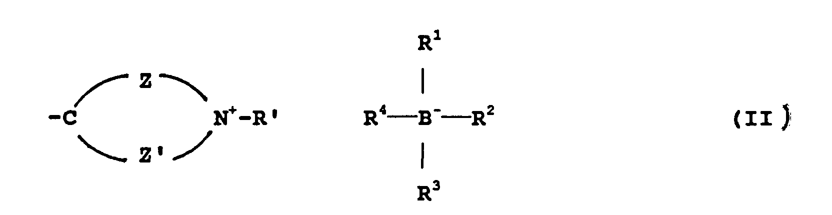
ist, worin
- R': eine (C₁-C₆)-Alkylgruppe oder eine Gruppe der Formel (CH₂)ₚ-CO-O-(C₁-C₆)-Alkyl, wobei p eine ganze Zahl von 1 bis 6 ist,
- R¹ - R⁴: unabhängig voneinander eine Alkyl-, Aryl-, Alkaryl-, Allyl-, Aralkyl-, Alkenyl- oder Alkynylgruppe oder eine gesättigte oder ungesättigte heterocyclische Gruppe, mit der Maßgabe, daß mindestens eine der Gruppen R¹ bis R⁴ eine (C₁-C₈)-Alkylgruppe ist,
- Z: eine Gruppe der Formel =CH-CH= und
- Z': eine Gruppe, die einen 5- oder 6-gliedrigen Ring vervollständigt, der als zweites Heteroatom ein Stickstoffatom, ein Sauerstoffatom oder ein Schwefelatom enthalten kann und gegebenenfalls mit einer (C₁-C₃)-Alkylgruppe oder einer Phenylgruppe substituiert oder mit einem Benzol- oder Naphthalinring anelliert ist, der seinerseits gegebenenfalls mit einem Halogenatom oder einer Phenylgruppe substituiert ist,
bedeuten.

Die Borat-Anionen sind an sich bekannt und von S. Murphy et al. in J. Org. Chem. 60 [1995] 2411 - 2422 beschrieben.

Als Halogenatome in Formel I sind Chloratome bevorzugt. Besonders bevorzugt sind Verbindungen, in denen darüber hinaus Q eine Trichlormethylgruppe ist. R' ist vorzugsweise eine Methyl-, Ethyl-, Hexyl- oder 3-Methoxycarbonylpropylgruppe. Bevorzugte Borat-Ionen in den Verbindungen der Formel I-IIb sind solche, in denen R¹, R² und R³ gleich sind und eine Phenyl- oder Anisylgruppe bedeuten und R⁴ eine n-Butylgruppe bedeutet.

Die Herstellung der erfindungsgemäßen Verbindungen erfolgt nach dem Fachmann prinzipiell bekannten Methoden.

Die Verbindungen der Formel I-II lassen sich durch Umsetzung eines Aldehyds der Formel OHC-[CH=CH]ₙ₋₁-A', wobei n die oben angegebene Bedeutung hat, und A' eine Gruppe der Formel

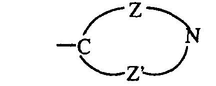

Bedeutet, worin Z und Z'die oben angegebene Bedeutung haben, mit einer Verbindung der Formel 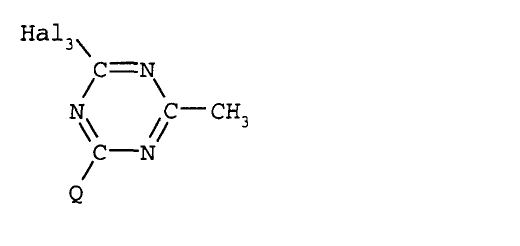 anschliessender

Behandlung des Reaktionsprodukts mit einem Halogenid der Formel R-Halogenid, worin R die oben angegebene Bedeutung hat, und abschließende Behandlung des sich dabei ergebenden Reaktionsprodukts mit einem Alkalimmetallborat der Formel 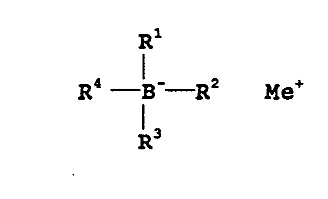 worin Me⁺ ein Alkalikation bedeutet, herstellen.

So läßt sich zum Beispiel eine Verbindung der Formel I-IIb, wie 1-Ethyl-4-[6-(4,6-bis-trichlormethyl-[1,3,5]-triazin-2-yl)-hexa-1,3,5-trienyl)-pyridinium-butyltriphenylborat, durch Überführung von Pyridin-4-carbaldehyd in 5-Pyridin-4-yl-penta-2,4-dienal, wie von M. Allasoued et al. beschrieben, erhalten. Diese Verbindung wird dann in einer Knoevenagel-Reaktion mit 2-Methyl-4,6-bis-trichlormethyl-[1,3,5]triazin zu 2-(6-Pyridin-4-yl-hexa-1,3,5-trienyl)-4,6-bis-trichlormethyl-[1,3,5]triazin umgesetzt. Schließlich wird noch der Pyridinstickstoff quaternisiert. Die Herstellung von 2-Methyl-4,6-bis-trichlormethyl-[1,3,5]triazin wurde von K. Wakabayashi et al. in Bl. Chem. Soc. Japan 42 [1969] 2924 - 2930 beschrieben.

Bei Bestrahlung, insbesondere mit Strahlung einer Wellenlänge von 330 bis 720 nm, setzen die erfindungsgemäßen Verbindungen Radikale frei. Somit lassen sie sich zur Induktion der Polymerisation oder Vernetzung ethylenisch ungesättigter Verbindungen einsetzen. Als ethylenisch ungesättigte Verbindungen kommen Monomere, Oligomere oder Polymere in Frage. Als Beispiele sind Polyvinylzimtsäureester, Hydroxy(meth)-acrylsäureester wie (Meth)acrylsäure-(3-hydroxypropyl)-ester oder (Meth)-acrylsäure-(4-hydroxybutyl)ester, 1,3-Di(meth)acryloylbutan, Pentaerythrittri(meth)acrylat, 2,2-Dimethylpropan-1,3-diol-(meth)acrylat, Polyethylen-glykolmono-(meth)acrylate, Polypropylenglykolmono(meth)-acrylate und Poly(ethylenglykol/propylenglykol)-mono-(meth)acrylate zu nennen. Die Oligomere weisen vorzugsweise ein Molekulargewicht M_{w} von 1500 bis 3500 auf. Als Polymere eignen sich Copolymere aus Methacrylsäureester und Methacrylsäure, Copolymere aus Styrol und Maleinsäureanhydrid, Terpolymere aus Acrylsäureethylester, Methacrylsäuremethylester und Acrylsäure, mit Isocyanat modifizierte Acrylsäureester, Methacrylsäureester und Itaconsäureester mehrwertiger Alkohole sowie Vinylurethan-Präpolymere.

Die mit den erfindungsgemäßen Verbindungen hergestellten strahlungsempfindlichen Gemische eignen sich als härtbare Beschichtungen im Bereich der Photographie, bei der Herstellung von vorsensibilisierten Druckplatten, Leiterplatten, Photoresists und Photomasken, Schwarzweißund Farbtransferfolien sowie Farbfolien. Außerdem sind sie als Photopolymerisationsinitiatoren in Zahnfüllungen, Zahnversiegelungen und dergleichen einsetzbar. Die Erfindung wird nun anhand der folgenden Beispiele näher erläutert.

### Synthese der Ausgangsmaterialien:

### Herstellung von tert.-Butyl-(2,2-bis-trimethylsilanylethyliden)-amin:

a) 132.15 g (3 mol) Acetaldehyd und 219.42 g (3 mol) tert.-Butylamin wurden unter Rühren gemischt, wobei die Temperatur auf 0°C gehalten wurde. Anschließend wurde wäßrige KOH zugegeben und die Mischung stehengelassen, bis sich zwei flüssige Phasen ausgebildet hatten. Die organische Schicht wurde abgetrennt und über KOH destilliert. Bei einem Siedepunkt (Kp.) von 85°C destillierten 267 g tert.-Butyl-ethyliden-amin (= H₃C-CH=N-C(CH₃)₃) über (Ausbeute: 90%).
b) 1000 ml einer 2M Lösung von n-Butyllithium in Pentan wurden unter Argon zu einer auf -20°C gehaltenen Mischung von 282 ml (2,2 mol) Diisopropylamin in 600 ml wasserfreiem Tetrahydrofuran (THF; durch Destillation über Natrium/Benzophenon gereinigt) gegeben. Zu dem auf -60°C gekühlten Gemisch wurde dann unter Rühren eine Mischung aus 100 g (1 mol) tert.-Butyl-ethyliden-amin in 200 ml wasserfreiem THF getropft. Nach 20 Stunden bei -60°C wurden 130 ml (1,1 mol) Chlortrimethylsilan zugegeben. Nach weiteren 5 Stunden bei -60°C wurden 134,9 g (1,1 mol) Chlortrimethylsilan zugegeben. Man ließ das Reaktionsgemisch langsam auf Raumtemperatur erwärmen. Der gebildete Niederschlag wurde entfernt und das Lösungsmittel bei verringertem Druck abgezogen. Schließlich wurde der verbleibende Rückstand im Vakuum destilliert. Es wurden 150 g tert.-Butyl-(2,2-bis-trimethyl-silanyl-ethyliden)-amin (Kp. 95°C/20 mmHg) erhalten (Ausbeute: 50%).

### Herstellung von 2-Methyl-4,6-bis-trichlormethyl-[1,3,5]-triazin:

Zu einer Mischung von 300 ml (3 mol) Trichloracetonitril und 96 ml (1,9 mol) Acetonitril wurden bei -10°C unter Rühren 4,5 g wasserfreies AlBr₃ gegeben. HCl-Gas wurde bis zur Sättigung eingeleitet. Nach 5 Stunden bei -10°C wurde auf 25,5°C erwärmt und weitere 17 h bei dieser Temperatur gehalten. Der so erhaltene Feststoff wurde durch Erwärmen auf 95°C geschmolzen und in 3000 ml kaltes Wasser gegossen. Der Niederschlag wurde abfiltriert, an der Luft getrocknet und umkristallisiert (aus Ethanol), was 400 g 2-Methyl-4,6-bis-trichlormethyl-[1,3,5]triazin (Schmelzpunkt: 96-97 °C, λₘₐₓ (Methanol): 219 nm, ∈ 1920) ergab.

### Herstellung von 5-Chinolin-4-yl-penta-2,4-dienal:

a) Zu einer Lösung von 7,53 g (48 mmol) Chinolin-4-carbaldehyd und 1,08 g (48 mmol) ZnBr₂ in 50 ml THF wurde bei Raumtemperatur unter Rühren eine Lösung von 12,85 g (52,8 mmol) tert.-Butyl-(2,2-bis-trimethylsilanyl-ethyliden)-amin in 25 ml THF gegeben. Nach 3 Stunden bei Raumtemperatur wurde eine Lösung von 10 g ZnCl₂ in einer Mischung von 100 ml Wasser, 125 ml Diethylether und 200 ml Essigsäureethylester zugegeben. Die Mischung wurde dann 2 Stunden bei Raumtemperatur stehengelassen. Es bildete sich eine feste Phase, die nun abgetrennt wurde. Das Lösungsmittel wurde von der flüssigen Phase so lange abgezogen, bis ein Niederschlag vorhanden war. Zu diesem wurde Essigsäureethylester gegeben, bis sich zwei Phasen gebildet hatten. Die organische Phase wurde dann über Na₂SO₄ getrocknet und das Lösungsmittel nochmals abgezogen. Der Rückstand bestand aus 42,8 mmol 3-Chinolin-4-yl-propenal (Ausbeute: 89,2%).
b) Zu einer Lösung von 3,9 g (21,3 mmol) 3-Chinolin-4-yl-propenal und 0,48 g (21,3 mmol) ZnBr₂ in THF wurde bei Raumtemperatur unter Rühren eine Lösung von 5,7 g (23,5 mmol) tert.-Butyl-(2,2-bis-trimethylsilanyl-ethyliden)-amin in 10 ml THF gegeben. Nach 4 h wurde eine Lösung von 4,3 g ZnCl₂ in einer Mischung aus 50 ml Wasser, 55 ml Diethylether und 100 ml Essigsäureethylester zugegeben. Nach weiteren 2 h wurde der gebildete Feststoff abfiltriert und die flüssige Phase wiederum bis zur Bildung eines Niederschlags aufkonzentriert. Zu diesem wurde Essigsäureethyl-ester gegeben, bis sich zwei Phasen gebildet hatten. Die organische Phase wurde dann über Na₂SO₄ getrocknet und das Lösungsmittel bei vermindertem Druck abgezogen. Der Rückstand bestand aus 17,3 mmol 5-Chinolin-4-yl-penta-2,4-dienal (Ausbeute: 81,4%).

Die folgenden Ausgangsmaterialien wurden analog zum obengenannten 3-Chinolin-4-yl-propenal erhalten (die bei jeder Reaktion eingesetzten molaren Mengen waren die gleichen wie dort angegeben).
3-Benzothiazol-2-yl-propenal (aus Benzothiazol-2-carbaldehyd), Ausbeute: 89%
3-Naphtho[2,3-d]thiazol-2-yl-propenal (aus Naphtho[2,3-d]thiazol-2-carbaldehyd), Ausbeute: 90%
3-Chinolin-2-yl-propenal (aus Chinolin-2-carbaldehyd), Ausbeute: 88%
3-(3,3-Dimethyl-3H-benzo[f]indol-2-yl)-propenal (aus 3,3-Dimethyl-3H-benzo[f]indol-2-carbaldehyd), Ausbeute: 91%
3-(6-Methyl-2-phenyl-pyrimidin-4-yl)-propenal (aus 6-Methyl-2-phenyl-pyrimidin-4-carbaldehyd), Ausbeute: 90%
3-(4-Phenyl-[1,3]-thiazol-2-yl)propenal (aus 4-Phenyl-[1,3]thiazol-2-carbaldehyd), Ausbeute: 89%
3-(4,5-Dihydro-[1,3]thiazol-2-yl-propenal (aus 4,5-Dihydro-[1,3]thiazol-2-carbaldehyd), Ausbeute: 91%
3-(4-Methyl-[1,3]thiazol-2-yl)-propenal (aus 4-Methyl-[1,3]thiazol-2-carbaldehyd), Ausbeute: 88%
3-(4-Phenyl-pyridin-2-yl)-propenal (aus 4-Phenyl-pyridin-2-carbaldehyd), Ausbeute: 89,6%
3-(4,5-Dihydro-[1,3]oxazol-2-yl-propenal (aus 4,5-Dihydro-[1,3]oxazol-2-carbaldehyd), Ausbeute: 88%
3-(4-Methyl-[1,3]oxazol-2-yl)-propenal (aus 4-Methyl-[1,3]oxazol-2-carbaldehyd), Ausbeute: 90%
3-Benzooxazol-2-yl-propenal (aus Benzooxazol-2-carbaldehyd), Ausbeute: 87%
3-(3-Hydroxy-1,1-dioxo-1λ⁶-benzo[b]thiophen-2-yl)-propenal (aus 3-Hydroxy-1,1-dioxo-1λ⁶-benzo[b]thiophen-2-carbaldehyd), Ausbeute: 91%
3-(5,6-Dichlor-1-ethyl-benzimidazol-2-yl)-propenal (aus 5,6-Dichlor-1-ethyl-benzimidazol-2-carbaldehyd), Ausbeute: 89%
3-(5-Chlor-benzooxazol-2-yl)-propenal (aus 5-Chlorbenzooxazol-2-carbaldehyd), Ausbeute: 90%
3-Naphtho[1,2-d]oxazol-2-yl-propenal (aus Naphtho[1,2-d]oxazol-2-carbaldehyd), Ausbeute: 88%
3-(5-Phenyl-benzooxazol-2-yl)-propenal (aus 5-Phenylbenzooxazol-2-carbaldehyd), Ausbeute: 91%
3-Pyridin-4-yl-propenal (aus Pyridin-4-carbaldehyd), Ausbeute: 90%

### Beispiel 1

### 1-Ethyl-4-[6-(4,6-bis-trichlormethyl-[1,3,5]triazin-2-yl)-hexa-1,3,5-trienyl]-chinolinium-butyltriphenylborat

a) Eine Mischung aus 7,06 g (33,8 mmol) 5-Chinolin-4-yl-penta-2,4-dienal, 11,2 g (33, 8 mmol) 2-Methyl-4,6-bis-trichlormethyl-[1,3,5]triazin und 80 ml destilliertem Toluol wurden in einem Reaktionsbehälter mit Rückflußkühler, mechanischem Rührer und Thermometer vorgelegt. Dann wurden 1,6 ml (28 mmol) Essigsäure und 1,94 ml (19,1 mmol) Piperidin zugegeben und die erhaltene Mischung 4 h zum Rückfluß erhitzt. Anschließend wurde das Toluol bei vermindertem Druck abgezogen und der Rückstand mit kaltem Hexan gewaschen. Der nicht in Hexan lösliche Rückstand wurde in einer Mischung aus Petrolether und Ethylacetat (3:7) aufgenommen und über einer Silicagel-Säule gereinigt. Nach Umkristallisieren (CH₂Cl₂) und Entfärbung (mit Tierkohle) wurden 8,4 mmol 2-(6-Chinolin-4-yl-hexa-1,3,5-trienyl)-4,6-bis-trichlormethyl-[1,3,5]triazin erhalten (Ausbeute: 25%).
b) Zu einer Lösung von 7,4 mmol 2-(6-Chinolin-4-ylhexa-1,3,5-trienyl)-4,6-bis-trichlormethyl-[1,3,5]-triazin in 80 ml Toluol wurden 1,17 g (7,4 mmol) Iodethan gegeben. Das Toluol wurde ohne Erwärmen im Vakuum abgezogen. Es wurden 7,2 mmol 1-Ethyl-4-[6-(4,6-bis-trichlormethyl-[1,3,5]triazin-2-yl)-hexa-1,3,5-trienyl)-chinoliniumiodid erhalten (Ausbeute: 97%).
c) Eine Lösung von 7,0 mmol 1-Ethyl-4-[6-(4,6-bis-trichlormethyl-[1,3,5]triazin-2-yl)-hexa-1,3,5-trienyl)-chinoliniumiodid in 80 ml THF wurde mit 7,0 mmol Lithium-butyltriphenylborat (Li⁺ [n-C₄H₉B(C₆H₅)₃]⁻) versetzt. Nach 1 h wurde das Lösungsmittel bei vermindertem Druck abgezogen, das Produkt lithiumiodidfrei gewaschen und anschließend getrocknet. Auf diese Art wurden 5,6 g 1-Ethyl-4-[6-(4,6-bis-trichlormethyl-[1,3,5]triazin-2-yl)-hexa-1,3,5-trienyl]-chinolinium-butyltriphenylborat (C₂₂H₁₇Cl₆N₄·C₂₂H₂₄B) erhalten (Ausbeute: 97%).

### Beispiele 2 bis 13

Analog - durch Umsetzung äquimolarer Mengen an 2-Methyl-4,6-bis-trichlormethyl-[1,3,5]triazin und einem Aldehyd - wurden die folgenden Salze hergestellt; das Iodid des jeweiligen Zwischenprodukts wurde durch n-Butyltriphenyl-borat ersetzt.
2) 2-[4-(4,6-Bis-trichlormethyl-[1,3,5]triazin-2-yl)-buta-1,3-dienyl]-3-ethyl-3H-benzothiazoliumbutyltriphenylborat (C₁₈H₁₃Cl₆N₄S·C₂₂H₂₄B)
3) 2-[4-(4,6-Bis-trichlormethyl-[1,3,5]triazin-2-yl)-buta-1,3-dienyl]-3-(3-methoxycarbonylpropyl)-naphtho[2,3-d]thiazolium-butyltriphenylborat (C₂₅H₁₉Cl₆N₄O₂S·C₂₂H₂₄B)
4) 2-[4-(4,6-Bis-trichlormethyl-[1,3,5]triazin-2-yl)-buta-1,3-dienyl]-1-ethyl-chinolinium-butyltriphenylborat (C₂₀H₁₅Cl₆N₄·C₂₂H₂₄B)
5) 2-[4-(4,6-Bis-trichlormethyl-[1,3,5]triazin-2-yl)-buta-1,3-dienyl]-1,3,3-trimethyl-3H-benzo-[f] indolium-butyltriphenylborat (C₂₄H₁₉Cl₆N₄·C₂₂R₂₄B)
6) 4-[4-(4,6-Bis-trichlormethyl-[1,3,5]triazin-2-yl)-buta-1,3-dienyl]-3,6-dimethyl-2-phenylpyrimidinium-butyltriphenylborat (C₂₁H₁₆Cl₆N₄·C₂₂H₂₄B)
7) 2-[4-(4,6-Bis-trichlormethyl-[1,3,5]triazin-2-yl)-buta-1,3-dienyl]-3-methyl-4-phenyl-[1,3]thiazolium-butyltriphenylborat (C₁₉H₁₃Cl₆N₄S·C₂₂H₂₄B)
8) 2-[4-(4,6-Bis-trichlormethyl-[1,3,5]triazin-2-yl)-buta-1,3-dienyl]-3-ethyl-4,5-dihydro-[1,3]thiazolium-butyltriphenylborat (C₁₄H₁₃Cl₆N₄S·C₂₂H₂₄B)
9) 2-[4-(4,6-Bis-trichlormethyl-[1,3,5]triazin-2-yl)-buta-1,3-dienyl]-3-ethyl-4-methyl-[1,3]thiazolium-butyltriphenylborat (C₁₅H₁₃Cl₆N₄S · C₂₂H₂₄B)
10) 2-[4-(4,6-Bis-trichlormethyl-[1,3,5]triazin-2-yl)-buta-1,3-dienyl]-1-ethyl-4-phenyl-pyridiniumbutyltriphenylborat (C₂₂H₁₇Cl₆N₄S · C₂₂H₂₄B)
11) 2-[4-(4,6-Bis-trichlormethyl-[1,3,5]triazin-2-yl)-buta-1,3-dienyl]-3-ethyl-4,5-dihydro-[1,3]oxazolium-butyltriphenylborat (C₁₄H₁₃Cl₆N₄O·C₂₂H₂₄B)
12) 2-[4-(4,6-Bis-trichlormethyl-[1,3,5]triazin-2-yl)-buta-1,3-dienyl]-3-ethyl-4-methyl-[1,3]oxazolium-butyltriphenylborat (C₁₅H₁₃Cl₆N₄O·C₂₂H₂₄B)
13) 2-[4-(4,6-Bis-trichlormethyl-[1,3,5]triazin-2-yl)-buta-1,3-dienyl]-3-hexyl-benzooxazoliumbutyltriphenylborat (C₂₂H₂₁Cl₆N₄O·C₂₂H₂₄B)

## Patentansprüche

1. Verbindungen der Formel I 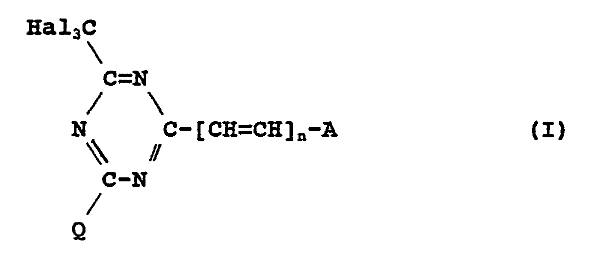 worin
n eine ganze Zahl von 1 bis 3,
Hal ein Halogenatom,
Q CCl₃, CBr₃, NH₂, NHR, NRR' oder OR, wobei R und R' unabhängig voneinander eine (C₁-C₆)-Alkylgruppe oder eine Phenylgruppe und
A ein Rest der folgenden Formel 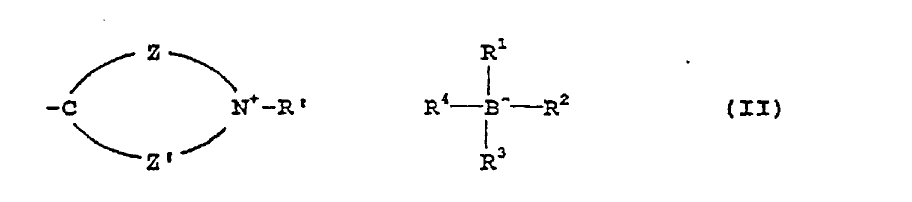
ist, worin
R' eine (C₁-C₆)-Alkylgruppe oder eine Gruppe der Formel (CH₂)ₚ-CO-O-(C₁-C₆)-Alkyl, wobei p eine ganze Zahl von 1 bis 6 ist,
R¹ - R⁴ unabhängig voneinander eine Alkyl-, Aryl-, Alkaryl-, Allyl-, Aralkyl-, Alkenyl- oder Alkynylgruppe oder eine gesättigte oder ungesättigte heterocyclische Gruppe, mit der Maßgabe, daß mindestens eine der Gruppen R¹ bis R⁴ eine (C₁-C₈)-Alkylgruppe ist,
Z eine Gruppe der Formel =CH-CH= und
Z' eine Gruppe, die einen 5- oder 6-gliedrigen Ring vervollständigt, der als zweites Heteroatom ein Stickstoffatom, ein Sauerstoffatom oder ein Schwefelatom enthalten kann und gegebenenfalls mit einer (C₁-C₃)-Alkylgruppe oder einer Phenylgruppe substituiert oder mit einem Benzol- oder Naphthalinring anelliert ist, der seinerseits gegebenenfalls mit einem Halogenatom oder einer Phenylgruppe substituiert ist,
bedeuten.

2. Verbindungen nach Anspruch 1, wobei Hal ein Chloratom bedeutet.

3. Verbindungen nach Anspruch 1 oder 2, wobei Q eine CCl₃-Gruppe bedeutet.

4. Verbindungen nach einem der Ansprüche 1 bis 3, wobei R' eine Methyl-, Ethyl-, Hexyl- oder 3-Methoxycarbonylpropylgryppe bedeutet.

5. Verfahren nach einem der Ansprüche 1, 2, 3 oder 4, wobei R¹, R² und R³ eine Phenyl- oder Anisylgruppe und R⁴ eine n-Butylgruppe bedeuten.

6. Verfahren zur Herstellung von Verbindungen der Formel I-II nach einem der Ansprüche 1, 2, 3, 4 oder 5, bei dem man ein Aldehyd der Formel OHC-[CH=CH]ₙ₋₁-A', worin
n die im Anspruch 1 angegebenen Bedeutung hat und
A'eine Gruppe der Formel 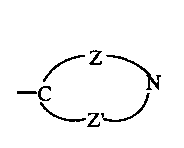 bedeutet, worin Z und Z'auch die oben angegebene Bedeutung haben, mit einem Triazinderivat der formel 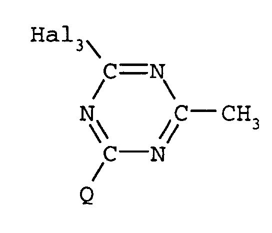 umsetzt und das Reaktionsprodukt anschliessend mit einem Halogenid der Formel R-Halogenid, worin R die in Anspruch 1 angegebene Bedeutung hat, unsetzt und es schliesslich mit einem Alkalimetallborat der Formel 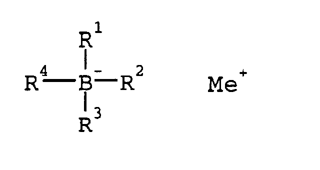 worin Me⁺ ein Alkalikation bedeutet, umsetzt.

## Claims

1. Compounds of the formula I 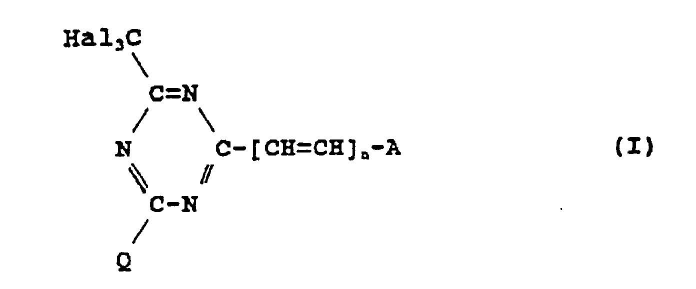 in which
n is an integer from 1 to 3,
Hal is a halogen atom,
Q is CCl₃, CBr₃, NH₂, NHR, NRR' or OR, where R and R', independently of one another, are a (C₁-C₆)-alkyl group or a phenyl group, and
A is a radical of the following formula 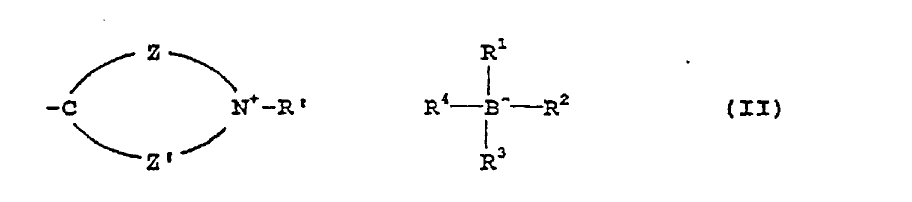
in which
R' is a (C₁-C₆)-alkyl group or a group of the formula (CH₂)ₚ-CO-O-(C₁-C₆)-alkyl, where p is an integer from 1 to 6,
R¹ - R⁴, independently of one another, are an alkyl, aryl, alkaryl, allyl, aralkyl, alkenyl or alkynyl group or a saturated or unsaturated heterocyclic group, with the proviso that at least one of the groups R¹ to R⁴ is a (C₁-C₈)-alkyl group,
Z is a group of the formula =CH-CH=, and
Z' is a group which completes a 5- or 6-membered ring, which may contain a nitrogen atom, an oxygen atom or a sulphur atom as second hetero atom and is optionally substituted by a (C₁-C₃)-alkyl group or a phenyl group or fused to a benzene or naphthalene ring, which is itself optionally substituted by a halogen atom or a phenyl group.

2. Compounds according to Claim 1, in which Hal is a chlorine atom.

3. Compounds according to Claim 1 or 2, in which Q is a CCl₃ group.

4. Compounds according to one of Claims 1 to 3, in which R' is a methyl, ethyl, hexyl or 3-methoxycarbonylpropyl group.

5. Process according to one of Claims 1, 2, 3 or 4, in which R¹, R² and R³ are a phenyl or anisyl group, and R⁴ is an n-butyl group.

6. Process for the preparation of compounds of the formula I-II according to one of the Claims 1, 2, 3, 4 or 5, in which an aldehyde of the formula OHC-[CH=CH]ₙ₋₁-A', in which
n is as defined in Claim 1 and
A' represents a group of the formula 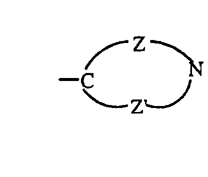 in which Z and Z' are also as defined above,
is reacted with a triazine derivative of the formula 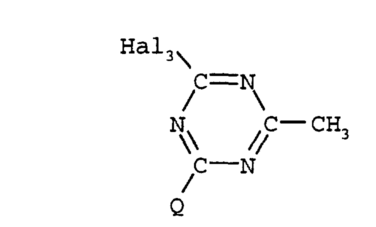 and the reaction product is subsequently reacted with a halide of the formula R-halide, in which R is as defined in Claim 1, and finally reacted with an alkali metal borate of the formula 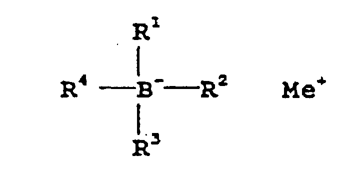 in which Me⁺ is an alkali metal cation.

## Revendications

1. Composés répondant à la formule I 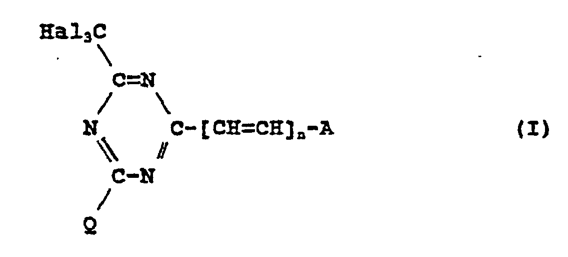 dans laquelle
n représente un nombre entier de 1 à 3,
Hal représente un atome d'halogène,
Q représente un groupe CCl₃, un groupe CBr₃, un groupe NH₂, un groupe NHR, un groupe NRR' ou un groupe OR, dans lesquels R et R' représentent indépendamment l'un de l'autre un groupe alkyle en C₁-C₆ ou un groupe phényle, et
A représente un radical répondant à la formule indiquée ci-après 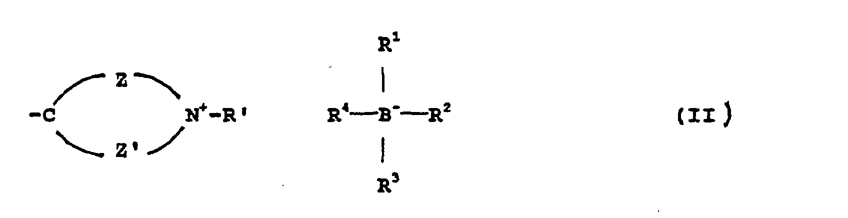
dans laquelle
R' représente un groupe alkyle en C₁-C₆ ou un groupe répondant à la formule (CH₂)ₚ-CO-O-alkyle en C₁-C₆ dans laquelle p représente un nombre entier de 1 à 6,
R¹ - R⁴ représentent indépendamment l'un de l'autre un groupe alkyle, un groupe aryle, un groupe alkaryle, un groupe allyle, un groupe aralkyle, un groupe alcényle ou un groupe alcynyle ou encore un groupe hétérocyclique saturé ou insaturé, avec cette mesure qu'au moins un des groupes R¹ - R⁴ représente un groupe alkyle en C₁-C₈,
Z représente un groupe répondant à la formule =CH-CH= et
Z' représente un groupe qui complète un noyau pentagonal ou hexagonal, ce dernier pouvant contenir, à titre d'hétéro-atome supplémentaire, un atome d'azote, un atome d'oxygène ou un atome de soufre et étant le cas échéant substitué avec un groupe alkyle en C₁-C₃ ou avec un groupe phényle ou encore étant condensé avec un noyau benzénique ou avec un noyau naphtalénique qui, à son tour, est le cas échéant substitué avec un atome d'halogène ou avec un groupe phényle.

2. Composés selon la revendication 1, dans lesquels Hal représente un atome de chlore.

3. Composés selon la revendication 1 ou 2, dans lesquels Q représente un groupe CCl₃.

4. Composés selon l'une quelconque des revendications 1 à 3, dans lesquels R' représente un groupe méthyle, un groupe éthyle, un groupe hexyle ou un groupe 3-méthoxycarbonylpropyle.

5. Procédé selon l'une quelconque des revendications 1, 2, 3 ou 4, dans lequel R¹, R² et R³ représentent un groupe phényle ou un groupe anisyle et R⁴ représente un groupe n-butyle.

6. Procédé pour la préparation de composés répondant aux formules I - II selon l'une quelconque des revendications 1, 2, 3, 4 ou 5, dans lequel on fait réagir un aldéhyde répondant à la formule OHC-[CH=CH]ₙ₋₁-A' dans laquelle
n a la signification indiquée à la revendication 1, et
A' représente un groupe répondant à la formule 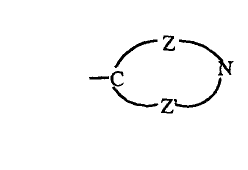
dans laquelle Z et Z' ont les significations indiquées ci-dessus, avec un dérivé de triazine répondant à la formule 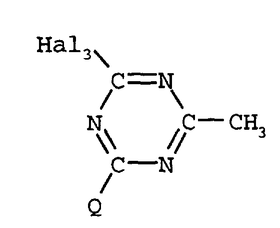 et on fait ensuite réagir le produit réactionnel avec un halogénure répondant à la formule R-halogénure dans laquelle R a la signification indiquée à la revendication 1, et on fait enfin réagir ce dernier avec un borate de métal alcalin répondant à la formule 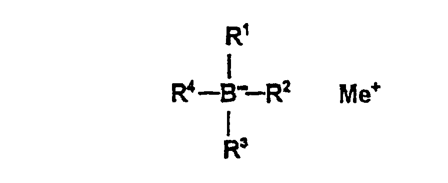 dans laquelle Me⁺ représente un cation de métal alcalin.
